# EUROPEAN PATENT APPLICATION

(11) **EP 4 697 336 A1**
(43) Date of publication of application: **18.02.2026**
(21) Application number: 25194860.0
(22) Date of filing: 08.08.2025
(51) Int. Cl.: G16C 20/20, G16C 20/70, H01J 49/00

(54) **MACHINE LEARNING SYSTEM FOR ANALYTE IDENTIFICATION**

(30) Priority: 12.08.2024 US 202418800969
(71) Applicant: HighChem s.r.o., 821 09 Bratislava (SK); Invitrogen BioServices India Private Limited, Bengaluru 560066 (IN); Thermo Fisher Scientific S.p.A, 20090 Rodano (MI) (IT)
(72) Inventor: RAAB, Michal, 82109 Bratislava (SK); WADINGER, Marek, 82109 Bratislava (SK); FALAQ, Maria, 560066 Bengaluru (IN); ULASZEWSKA, Marynka, 20090 Rodano (IT)
(74) Representative: Boult Wade Tennant LLP

(57) **Abstract**

An example method performed via a computing device for providing support to a mass spectrometry (MS) system includes obtaining from a mass spectral library (i) an ordered hitlist of reference spectra corresponding to a set of fragmentation spectra of an analyte acquired with the MS system; and (ii) a first set of metadata corresponding to the ordered hitlist of reference spectra. The method also includes obtaining from the MS system a second set of metadata corresponding to the set of fragmentation spectra. The method also includes evaluating an order of entries in the ordered hitlist with a machine learning model based on the first set of metadata and the second set of metadata.

## Description

### TECHNICAL FIELD

Various examples relate generally, but not exclusively, to support systems for scientific instruments, such as mass spectrometry systems.

### BACKGROUND

Determining the identity of a compound is one of the main tasks in chemical analysis. For compounds in complex mixtures, fragmentation patterns of their ions can be used for this purpose. The corresponding mass spectra can provide both the elemental composition of the compound and a direct read-out of labile bonds. When combined with gas or liquid chromatography (GC or LC), mass spectrometry (MS) can distinguish hundreds of components in complex mixtures.

Steady advances in the sensitivity and resolution of mass spectrometers continue to provide new capabilities for detecting ever-increasing numbers of components in chemical mixtures. Dealing with the increasing number of identifiable compounds and associated digital data presents a significant challenge to the effective use of such advanced instruments. An example tool for analyzing high-resolution mass spectrometry (HR-MS) data includes the use of spectral libraries: collections of chemical structures and their mass spectra that can support fast, reliable identification of a compound whose fragmentation pattern is measured with the MS instrument.

### SUMMARY

Disclosed herein are, among other things, various examples, aspects, features, and embodiments of a machine learning (ML) system for analyte identification. In one example, an ML classification algorithm for small molecule identification presents a modified scoring and ranking system that considers certain metadata of the query spectra and of the corresponding mass spectral library matches. Such metadata may include, but are not limited to, the normalized collision energy, ion activation method parameters, and/or pertinent characteristics of the candidate compound pre-selection. In at least some examples, the modified scoring and ranking system beneficially enables the corresponding ML system to generate modified hitlists having a higher proportion of correct candidates at the top thereof due to the combined use of similarity scoring and data-driven model stacking.

One example provides a method performed via a computing device for providing support to an MS system, the method comprising: obtaining from a mass spectral library (i) an ordered hitlist of reference spectra corresponding to a set of fragmentation spectra of an analyte acquired with the MS system; and (ii) a first set of metadata corresponding to the ordered hitlist of reference spectra; obtaining from the MS system a second set of metadata corresponding to the set of fragmentation spectra; and evaluating an order of entries in the ordered hitlist with an ML model based on the first set of metadata and the second set of metadata.

Another example provides an apparatus for providing support to an MS system, the apparatus comprising: an interface device; a processing device; and a memory device including program code, wherein the memory device and the program code are configured to, with the interface device and the processing device, cause the apparatus at least to: obtain from a mass spectral library (i) an ordered hitlist of reference spectra corresponding to a set of fragmentation spectra of an analyte acquired with the MS system; and (ii) a first set of metadata corresponding to the ordered hitlist of reference spectra; obtain from the MS system a second set of metadata corresponding to the set of fragmentation spectra; and evaluate an order of entries in the ordered hitlist with an ML model based on the first set of metadata and the second set of metadata.

### BRIEF DESCRIPTION OF THE DRAWINGS

The foregoing aspects and many of the attendant advantages of the present disclosure will become more readily appreciated as the same become better understood by reference to the following detailed description, when taken in conjunction with the accompanying drawings, in which:
FIG. 1 is a block diagram illustrating a machine learning (ML) system for analyte identification according to some examples.
FIG. 2 is a block diagram illustrating a model module used in the ML system of FIG. 1 according to one example.
FIG. 3 is a block diagram illustrating an encoder used in the model module of FIG. 2 according to one example.
FIG. 4 is a block diagram illustrating a workflow for preparing inputs to the encoder of FIG. 3 according to one example.
FIG. 5 is a block diagram illustrating a training process applied to the ML model used in the model module of FIG. 2 according to one example.
FIG. 6 is a flowchart illustrating a method for analyte identification implemented using the ML system of FIG. 1 according to some examples.
FIGS. 7A-7C graphically illustrate certain operations of the method of FIG. 6 according to one example.
FIG. 8 is a block diagram illustrating a computing device, one or more instances of which can be used in the ML system of FIG. 1, according to some examples.
FIG. 9 is a block diagram illustrating an MS instrument support system in which some or all of the MS instrument support methods and/or functions disclosed herein may be performed according to some examples.
FIGS. 10A-10B illustrate improvements achievable with the method of FIG. 6 over conventional analyte identification methods according to one example.

### DETAILED DESCRIPTION

Mass spectral libraries are an important resource to analytical chemists across a variety of applications. For example, the National Institute of Standards and Technology (NIST) provides several curated libraries of mass spectral reference data. Additionally, NIST produces and distributes search software for interacting with the libraries. Other mass spectral libraries and the corresponding search software for interacting with those libraries are available from various additional providers including, but not limited to, mzCloud, Mass Frontier, and myLibrary.

A typical mass spectral library search algorithm calculates a matching score (also sometimes referred to as the match factor) between a query spectrum and a set of reference spectra. In some examples, the matching score is represented by an integer between 0 and 999 that quantifies the "similarity" between a pair of spectra. Different search algorithms usually differ in how they compute the matching scores. In different search configurations, the set of reference spectra may include the entire library of spectra (i.e., no pre-search is performed) or a selected subset of the library spectra identified during preselection or pre-search. The search algorithm typically returns a list of reference spectra sorted in the descending order of the matching score. The returned ordered list is often referred to as the "hitlist" of the query.

Three commonly employed algorithms implemented in NIST MS Search software are the normal-identity, simple-similarity, and hybrid-similarity searches. Each of these algorithms uses some or all of the following basic operations: pre-search, peak matching, dot-product calculation, matching score calculation, and hitlist ranking and display. When used, pre-searching selects a subset of the library spectra likely to score highly. The objective of the normal-identity search algorithm is to return a hitlist that contains the correct identification of the query spectrum, preferably at the top of the hitlist. The objective of both the "simple" and "hybrid" similarity searches is to return a hitlist that can help an analyst to propose a structure for their query compound (analyte).

Whenever an expert analyst reviews a hitlist, they can often pinpoint certain weaknesses in the putative identification of the analyte. One example weakness might occur when the respective experimental conditions of the reference spectrum and query do not sufficiently match. Another example weakness might occur when there are too few peaks for sufficiently confident identification, etc. Some embodiments disclosed herein are directed at replacing the manual, human-centric "expert analyses" of the hitlist by a machine learning (ML) model that can increase confidence in the identification workflow. In some examples, the disclosed ML system for analyte identification can beneficially achieve higher specificity (e.g., through reducing the number of false positives) compared to algorithms relying on the metrics derived mostly from the spectrum similarity determined based on the above-mentioned dot-product metrics and the like.

According to one example, an ML classification algorithm for small molecule identification presents a modified scoring and ranking system that considers annotations and metadata of the query and library-spectra matches. On top of the similarity metric between the unknown and candidate spectra, the ML classification algorithm employing a suitably selected classifier, such as, for example, Random Forest, Logistic Regression, Bayesian Network, XGBOOST, LightGBM and the like, takes into consideration certain metadata, such as the normalized collision energy, ion activation method parameters, and pertinent characteristics of the candidate compound pre-selection (such as the number of candidate compounds, average spectral matching score, and others). In some examples, the side information associated with the fragmentation spectra that is fed into the ML classification algorithm may include one or more of the following: precursor ion m/z, precursor ion formula, charge state, peaks count, peak sparseness, peak accuracy, peak intensity, peak distances, neutral losses, peaks breakdown curve, peaks formula, peaks structure, chemical class, compound class and one or more mean, median, average, standard deviations, standard errors, relative standard deviation, relative error or variance values thereof. In at least some examples, the modified scoring and ranking system beneficially enables the corresponding ML system to generate hitlists having a higher proportion of correct candidates at the top thereof.

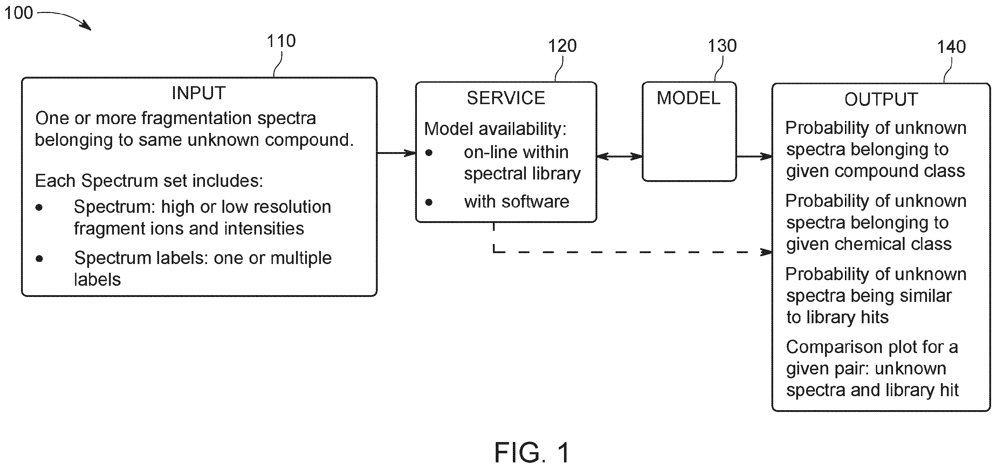

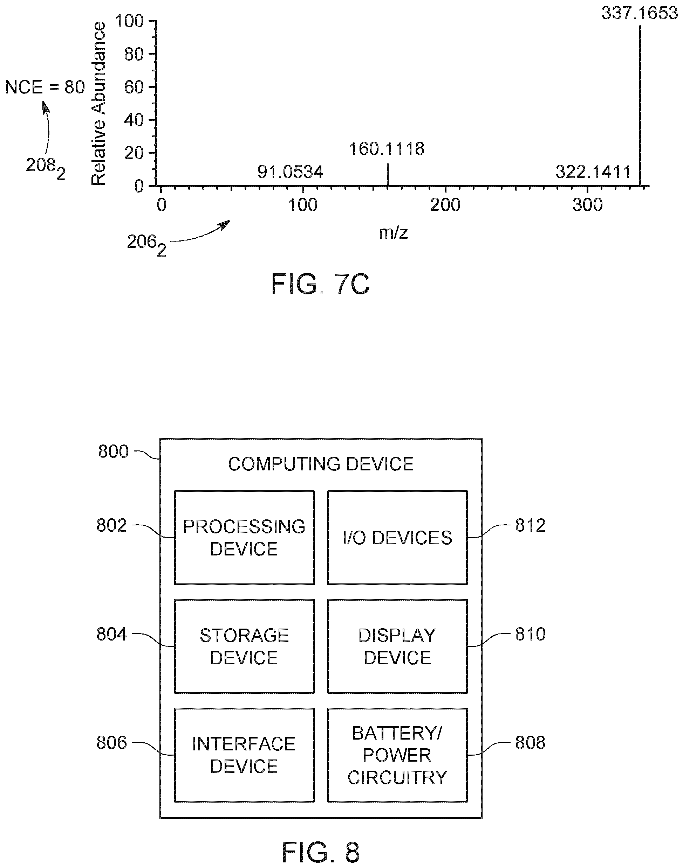

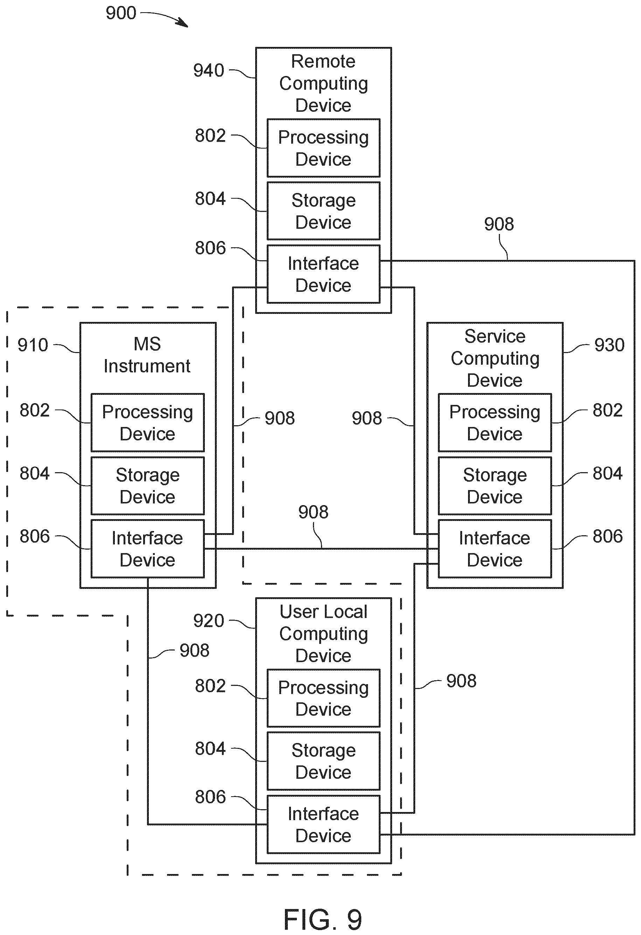

FIG. 1 is a block diagram illustrating an ML system 100 for analyte identification according to some examples. The system 100 may be implemented by circuitry (e.g., including electrical and/or optical components), such as one or more programmed computing devices. Examples of computing devices that may, singly or in combination, implement the ML system 100 are described in more detail below in reference to FIG. 8. Additionally, examples of systems of interconnected computing devices, to which the corresponding MS instrument is connected, are described in more detail below in reference to FIG. 9. In the example shown, the ML system 100 includes an input module 110, a service module 120, a model module 130, and an output module 140.

The input module 110 may be provided as part of a user interface through which search queries can be submitted for analyte identification. In one example, a search query submitted through the input module 110 includes one or more fragmentation mass spectra acquired with the corresponding MS instrument. The submitted mass spectra typically correspond to the same unknown compound. Each mass spectrum is a part of a spectrum dataset that typically includes pertinent metadata in addition to the mass spectrum itself. Herein, the term "mass spectrum" refers to a list of *(m*/*z, I*) data points, where *m, z,* and *I* are mass, charge, and intensity, respectively. The metadata may include one or more spectrum labels, a set of sample characteristics, and/or a set of acquisition parameters with which the spectrum was measured by the MS instrument. Various embodiments may accept one or more of: electron ionization (EI) spectra, small molecule tandem spectra, and peptide tandem spectra. EI searches are typically performed with unit-mass resolution, whereas tandem searches typically accept high-resolution spectra using either relative (ppm) or absolute (m/z) tolerances.

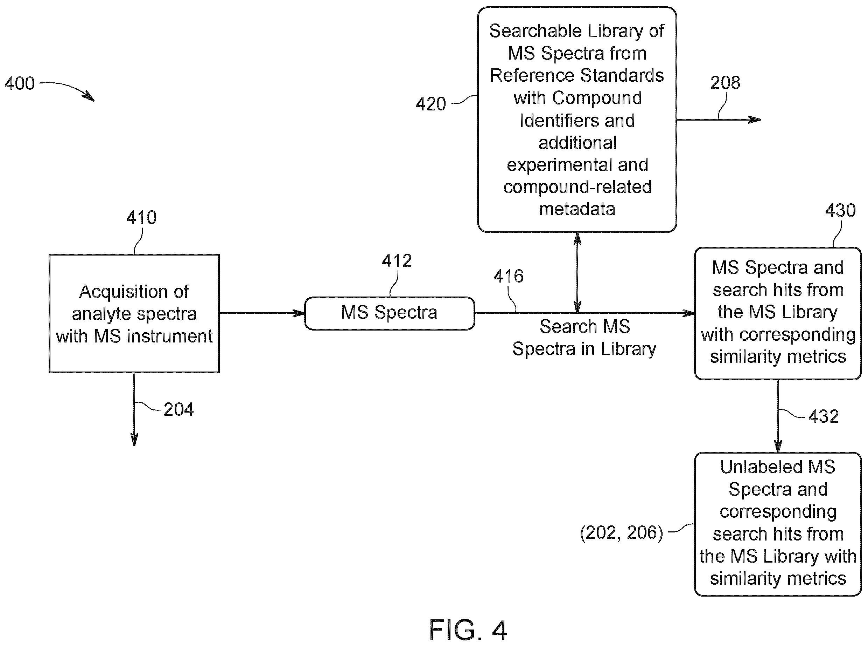

The service module 120 provides connectivity between the input module 110 and the model module 130. In some examples, the trained ML model provided via the model module 130 resides at the same server as the pertinent mass spectral library. In some other examples, the trained ML model provided via the model module 130 resides at a different network-connected server or at a local (to the user) computing device. In latter examples, the service module 120 also provides operational interconnectivity between the model module 130 and the mass spectral library (not explicitly shown, e.g., see FIG 4). In some examples, the service module 120 is configured to provide flexibility in data, library, and ML model access modes, e.g., with FaaS functions, various programming languages, "big data" solutions, and the like. Herein, the acronym "FaaS" stands for function as a service, which is a category of cloud computing services allowing customers to develop, run, and manage application functionalities without the complexity of building and maintaining the infrastructure typically associated with developing and launching an app.

In some examples, the trained ML model provided via the model module 130 is a random forest classifier. In some other examples, suitable alternatives to the random forest classifier can similarly be used. Example alternatives include a gradient boosting model, a k-Nearest Neighbors (kNN) algorithm, a variational autoencoder, and so on.

In some examples, the output module 140 is provided as part of the same user interface as the input module 110. In such examples, the service module 120 additionally provides operational connectivity between the model module 130 and the output module 140, e.g., as indicated in FIG. 1 by the dashed arrow. Example outputs generated with the trained ML model of the model module 130 and provided to the user via the output module 140 include one or more of: (i) the estimated probability of the compound characterized by the queried spectra belonging to a given compound class; (ii) the estimated probability of the compound characterized by the queried spectra belonging to a given chemical class; (iii) the estimated probabilities of the queried spectra being similar to some or all of the library spectra from the hitlist; and (iv) comparison plots for the queried spectra and the library spectra from the hitlist.

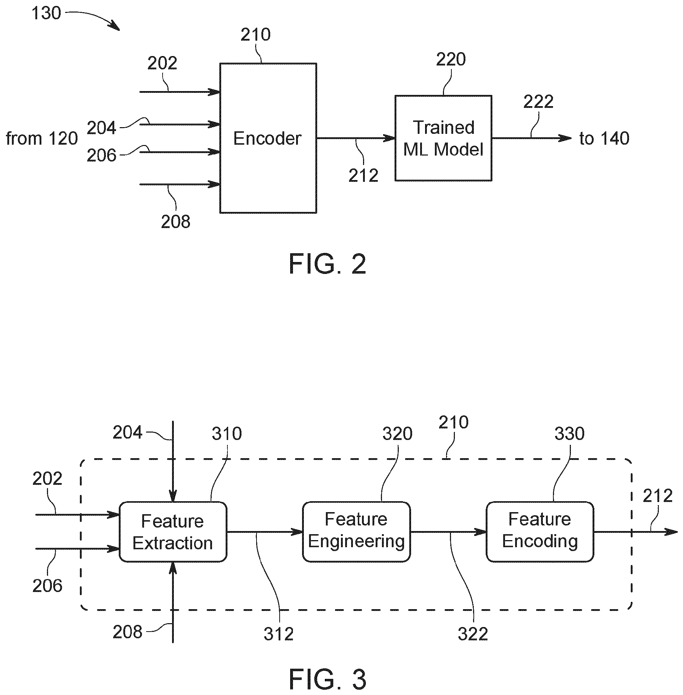

FIG. 2 is a block diagram illustrating the model module 130 according to one example. In the example shown, the model module 130 includes an encoder 210 and a trained ML model 220. The encoder 210 operates to transform the inputs 202, 204, 206, and 208 into a corresponding features vector 212. The features vector 212 is then processed with the trained ML model 220 to generate an output 222 for the output module 140.

In some examples, the inputs 202, 204, 206, and 208 are as follows. The input 202 has a set of mass spectra of the analyte acquired with the corresponding MS instrument. In some examples, such set includes a single spectrum. In some other examples, such set includes a plurality of spectra. The input 204 has a set of metadata corresponding to the spectra of the input 202. The input 206 has the hitlist spectra obtained from the mass spectral library via a conventional search for closest matches to the spectra of the input 202. The input 208 has a set of metadata corresponding to the hitlist spectra of the input 206 that is retrieved from the mass spectral library together with those spectra.

In one example, to accommodate the widely varying inputs 202, 204, 206, 208 for different analyte samples, the encoder 210 first performs tokenization configured to map the inputs 202, 204, 206, 208 to a corresponding token vector having a fixed length. The token vector is then further encoded by the encoder 210 to obtain the corresponding features vector 212. The features vector 212 can qualitatively be understood as containing the information about various features conveyed via the inputs 202, 204, 206, 208 in the form that can be leveraged to guide the generation of the output 222 in the trained ML model 220.

FIG. 3 is a block diagram illustrating the encoder 210 according to another example. In the example shown, the encoder 210 includes a feature extraction block 310, a feature engineering block 320, and a feature encoding block 330 serially connected as indicated in FIG. 3. The inputs 202, 204, 206, 208 are applied to the feature extraction block 310. The output 212 is generated by the feature encoding block 330.

In the feature extraction block 310, the inputs 202, 204, 206, and 208 are used to identify and extract relevant features from the raw data. The corresponding operations of the block 310 include applying data preparation techniques, such as scaling, missing value imputation, and separating mixed variables into separate features on the raw dataset for further processing. A first intermediate output 312 generated with the feature extraction block 310 is used in the feature engineering block 320 to create new features or to transform existing features. The corresponding operations of the block 320 may include: (i) for pairs of features, compute a first discrete difference of elements; and (ii) for individual features, computing a description of the set with respect to the group including, but not limited to, the rank, size, mean, standard deviation, and absolute deviation of the mean from raw numerical features as well as features based on relative abundance and matching peaks. A second intermediate output 322 generated by the feature engineering block 320 is further transformed in the feature encoding block 330. For example, certain ML algorithms work exclusively with numerical values and, as such, it may be advisable to transform categorical values of relevant features into numerical features, e.g., in the form of vector values. Consequently, in the block 330, certain categorical features, such as the types of analyzers, ion activation methods, and ionization methods, can be transformed from their respective categorical values into the relevant numerical features that can be processed by the ML model employed in the block 130. In some examples, the features vector 212 is additionally processed to make sure that only relevant features, such as the target label, are included therein, whereas outliers and missing values are not present in the dataset used for the training and validation of the ML model 220.

FIG. 4 is a block diagram illustrating a workflow 400 for preparing the inputs 202, 204, 206, 208 according to one example. The workflow 400 includes operating the corresponding MS instrument to acquire one or more MS spectra 412 of the analyte in question. The corresponding set of acquisition parameters for each of the acquired MS spectra 412 is exported from the MS instrument to form at least a portion of the input 204 to the encoder 210.

The workflow 400 also includes using the acquired MS spectra 412 to submit a corresponding search query to a mass spectral library 420 for performing a corresponding search 416 therein. The mass spectral library 420 has a collection of searchable reference spectra annotated with compound identifiers and further associated with the corresponding experimental and compound-related metadata. The search 416 returns a hitlist 430 including a subset of the reference spectra from the mass spectral library 420 and further including the corresponding similarity metrics with respect to the experimentally acquired MS spectra 412. In some examples, the similarity metrics used with the hitlist 430 include the above-mentioned matching scores. A set of experimental and compound-related metadata corresponding to the reference spectra of the hitlist 430 is also retrieved from the mass spectral library 420. In some examples, the mass spectral library 420 is the NIST library or the mzCloud library.

The workflow 400 optionally includes trimming or filtering the set of metadata retrieved from the mass spectral library 420 to remove parts of the metadata representing the features not used with the model module 130. Depending on the system embodiment and the specifics of the used mass spectral library 420, the original set of metadata retrieved from the mass spectral library 420 or the trimmed/filtered subset thereof is used to form the input 208 to the encoder 210.

The workflow 400 also includes applying one or more preprocessing operations 432 to the hitlist 430 and the associated MS spectra 412. Outputs generated with the preprocessing operations 432 are used to from the inputs 202 and 206 to the encoder 210. Examples of the preprocessing operations 432 include: removing the compound labels, removing at least some of the annotations, removing some library compounds from the hitlist based on structural similarity or dissimilarity, removing specific peaks from the hitlist, and the like.

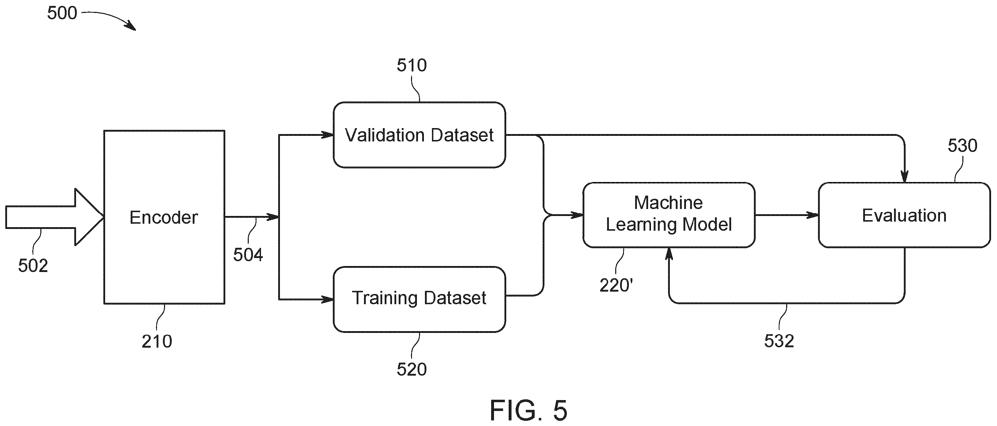

FIG. 5 is a block diagram illustrating a training process 500 used to train the ML model 220 (FIG. 2) according to one example. In general, the ML-model training process depends on the type of the model. In the example shown, the training process 500 corresponds to an embodiment in which the ML model 220 is a random forest classifier. Based on the provided description, a person of ordinary skill in the pertinent art will be able to implement other training processes suitable for other model types, without any undue experimentation.

A random forest classifier includes a plurality of decision trees, each of which outputs a respective prediction. When performing a classification task, each decision tree in the random forest votes for one of the classes to which the input may belong. After all of the trees have voted, the random forest classifier counts which class has the most populous vote, and this class is what the random forest classifier outputs as a final prediction. An individual decision tree splits data into groups of data based on the features represented by the data. The decision tree will continue to split the data into groups until a small set of data under one label (class representation) remains. The decision tree determines where to split the represented features based on a purity measure that measures information gain. For the classification task, the decision tree makes that decision based on the Gini index or entropy, and in the case of regression, based on the residual sum of squares.

The random forest logic can briefly be described as follows:
i. Suppose the number of observations *is N.* These *N* observations will be sampled at random with replacement.
ii. Suppose there are *M* features in the observations. A number *m,* where *m < M,* will be selected at random at each node from the total number of features, *M*. The best split on these m variables is used to split the node, and this value typically remains constant as the forest grows.
iii. Each decision tree in the forest is grown to its largest extent.
iv. The forest will output a prediction based on the aggregated predictions of the trees in the forest. In different examples, the aggregation can be either the majority vote or average.

The training process 500 applied to the random forest classifier 220' uses a volume 502 of reference MS data with the corresponding experimental-conditions and compound-related metadata. The volume 502 is encoded with the encoder 210 into a corresponding plurality 504 of features vectors 212 for which the ground truth classification is known. The plurality 504 is then split into a validation dataset 510 and a training dataset 520. The training dataset 520 is used to train the random forest classifier 220', while the validation dataset 510 is used to evaluate the classifier's performance. In one example, 75% of the plurality 504 is used for training, and the remaining 25% is used for evaluation. In other examples, other splitting ratios can also be used. Herein, the notation 220' indicates that the random forest classifier is not fully trained yet. As indicated above, the corresponding fully trained random forest classifier is denoted with the reference numeral 220 (e.g., see FIG. 2).

When properly configured and trained, the random forest classifier 220 should perform approximately equally on both of the datasets 510 and 520. The training process 500 includes an evaluation module 530 configured to evaluate the relative performance of the random forest classifier 220' on the datasets 510 and 520. Based on the evaluation 530, adjustments 532 are applied to the random forest classifier 220'. The evaluations 530 and the adjustments 532 are iteratively repeated until the applicable training-stoppage criterion is met. At that point, the random forest classifier 220' is locked and is deemed to be fully trained and suitable for use in the model module 130 (FIG. 2). Example operations performed during the training process 500 may include some or all of the following: data exploration, exploratory data analysis (EDA), setting threshold values, splitting the training data into training and validation datasets, scaling the data representing different features to the same scale, instantiating the forest, making predictions, evaluating the classifier performance using a scoring method, evaluating the classifier performance using a confusion matrix, ranking the features in the order of importance, choosing the number of trees for the forest, choosing the metric(s) used to split the features vectors into data groups, tuning the forest parameters using a random grid search, creating a dictionary of values to choose from, and tuning the forest parameters using a grid search within a delimited parameter space.

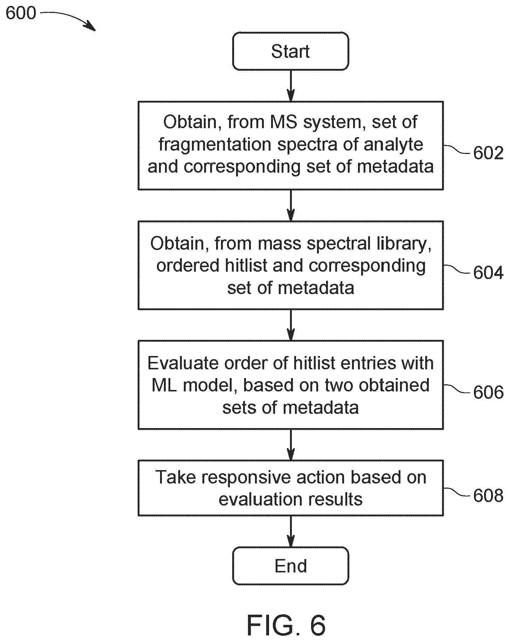

FIG. 6 is a flowchart illustrating a method 600 for analyte identification according to some examples. The method 600 can be implemented, e.g., using the ML system 100 of FIG. 1. The method 600 is described below in continued reference to FIGS. 1, 2, 4, and 6.

The method 600 includes the ML system 100 (FIG. 1) obtaining, from an MS system, a set of fragmentation spectra of an analyte and the corresponding set of metadata (in a block 602). In some examples, the set of fragmentation spectra is experimentally measured with the MS system. In some other examples, the set of fragmentation spectra is retrieved from a network-connected storage to which it was previously transferred from the MS system upon completion of the experimental acquisition. In various examples, the set of metadata obtained in the block 602 includes side information pertaining to the experimental conditions, system configuration, and analyte sample.

The method 600 also includes the ML system 100 obtaining, from the mass spectral library 420 (FIG. 4), an ordered hitlist and the corresponding set of metadata (in a block 604). In various examples, the ordered hitlist obtained in the block 604 includes reference spectra corresponding to the set of fragmentation spectra of the analyte obtained in the block 602. The hitlist is typically ordered in a descending order of the matching scores of the entries. In a typical example, a matching score is computed at the mass spectral library 420 using a spectrum similarity measure based on approaches, such as the dot product or Spearman's rank correlation coefficient of the corresponding pair of spectra. Operations of the block 604 typically include submitting to the mass spectral library 420, via the service module 120 (FIG. 1), a search query with the set of fragmentation spectra obtained in the block 602.

In various examples, the sets of metadata obtained in the blocks 602, 604 may include one or more of the following components: a normalized or absolute collision energy; one or more parameters of an ion activation method; a number of candidate compounds; one or more matching scores; an identity of a precursor ion; a precursor ion formula, a fragment ion formula, a number of peaks in a spectrum; a sparseness measure; spectrum peak counts, an intensity of a peak and a corresponding accuracy; one or more distances between peaks in a spectrum, neutral losses, peaks breakdown curves; one or more spectrum labels; a mean; a median; an average; standard deviations; standard errors; a relative standard deviation; relative errors; and/or a variance value of a selected numerical characteristic. In some examples, labels and parameters are grouped into subcategories including, but not limited to, query and library spectra metadata, constructed features metadata differences, score statistics (for example, rank, size, number of unique values, mean, standard deviation, z-score, absolute deviations) over different groupings (such as, a group by query spectrum, a group by query spectrum and library compound, a group by query spectrum and library compound), and a maximum score in the library compound group. The subcategories are then used to calculate the statistics per query spectrum. In some examples, the two sets of metadata may have the same composition of components. In some other examples, the two sets of metadata may have different respective compositions of components.

The method 600 also includes the ML system 100 evaluating the order of entries in the hitlist with the ML model 220 (in a block 606). In various examples, the evaluation is based, *inter alia,* on the two sets of metadata obtained in the blocks 602, 604. In one example, operations of the block 606 include: (i) with the encoder 210 (FIG. 2), generating the features vector 212 based on the set of fragmentation spectra of the analyte, reference spectra from the ordered hitlist, and two sets of metadata obtained in the blocks 602, 604; and (ii) applying the features vector 212 to the ML model 220 (FIG. 2). In response to the features vector 212, the ML model 220 may change the order of the hitlist entries in at least some cases. In such cases, the operations the block 606 may also include determining an adjustment value to a matching score provided by the mass spectral library 420 with the respective reference spectrum of the ordered hitlist.

In some examples, operations of the block 606 include the ML model 220 determining an estimated probability of the analyte belonging to a specified compound class, an estimated probability of the analyte belonging to a specified chemical class, and/or an estimated probability of the analyte being from a same compound class as a compound corresponding to a selected reference spectrum from the ordered hitlist received from the mass spectral library 420 in the block 604. In some cases, at least some of these estimated probabilities may differ from a corresponding probability determined at the mass spectral library 420.

The method 600 also includes the ML system 100 taking a responsive action (in a block 608). The taken responsive action is typically based on the evaluation results of the block 606. In one example, operations of the block 608 include displaying for a user, on a display device, a modified hitlist having a changed order of the entries therein. In another example, operations of the block 608 include notifying the user that the order of entries in the hitlist obtained in the block 604 remains unchanged. In yet another example, operations of the block 608 include suggesting a chemical identity of the analyte and displaying the corresponding molecular and structural information on the display device. In other examples, other responsive actions may also be taken in the block 608. Upon completion of the operations of the block 608, the method 600 is terminated.

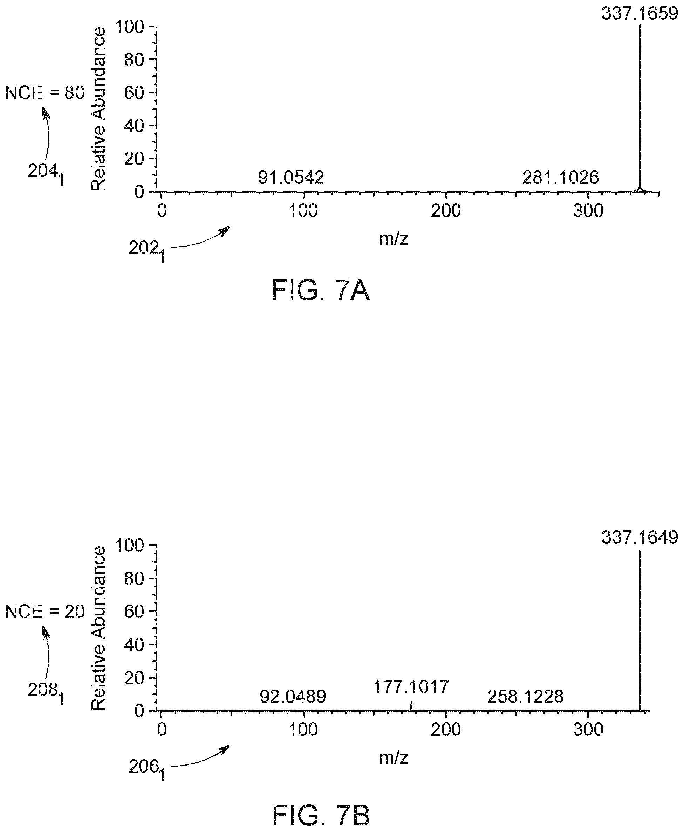

FIGS. 7A-7C graphically illustrate certain operations of the method 600 according to one example. More specifically, FIG. 7A graphically shows an MS spectrum 202₁ of an analyte in question acquired by operating the corresponding MS instrument. The normalized collision energy (NCE) value corresponding to that experimental run is NCE=80%. This NCE value is an example of the metadata 204 (FIG. 2) and, as such, is labeled in FIG. 7A using the reference numeral 204₁. FIGS. 7B and 7C graphically show hitlist spectra 206₁ and 206₂, respectively, obtained from the mass spectral library 420 via the search 416 (FIG. 4) for closest matches to the MS spectrum 202₁. The metadata corresponding to the hitlist spectra 206₁ and 206₂ that are retrieved from the mass spectral library 420 together with those spectra include the NCE values NCE=20% and NCE=80%, respectively. These NCE values are examples of the metadata 208 and, as such, are labeled in FIGS. 7B-7C using the reference numerals 208₁ and 208₂, respectively.

In the example shown, the mass spectral library 420 (FIG. 4) is configured to rank the hitlist entries based on the spectrum similarity metric, e.g., corresponding to approaches such as the cosine (dot product) metric, without considering the NCE values. According to that methodology, the spectrum 206₁ has the highest matching score among the hitlist entries. As such, the mass spectral library 420 ranks the spectrum 206₁ to be at the top of the hitlist. Accordingly, the spectrum 206₂ is listed lower in the hitlist outputted by the mass spectral library 420 than the spectrum 206₁.

Based on the above hitlist rankings, the chemical compound corresponding to the spectrum 206₁ may be predicted to be the same as or closest to the analyte in question. This however is an erroneous analyte identification that can beneficially be corrected with the ML system 100 (FIG. 1). More specifically, by also considering the NCE values 204₁, 208₁, and 208₂ as parts of the metadata 204 and 208, the model module 130 (FIGS. 1, 2) re-ranks the hitlist received from the mass spectral library 420. After such reranking, the resulting modified hitlist has the spectrum 206₂ at the top of the hitlist. Based on the modified hitlist rankings, the chemical compound corresponding to the spectrum 206₂ may be predicted to be the same as or closest to the analyte in question. In this particular example, the latter prediction provides the correct analyte identification.

FIG. 8 is a block diagram illustrating a computing device 800 one or more instances of which can be used in the ML system 100 according to some examples. In some examples, one instance of the computing device 800 is configured to implement the model module 130. In some examples, one or more instances of the computing device 800 can be used in the workflow 400, the process 500, and/or the method 600.

The computing device 800 of FIG. 8 is illustrated as having a number of components, but any one or more of these components may be omitted or duplicated, as suitable for the application and setting. In some embodiments, some or all of the components included in the computing device 800 may be attached to one or more motherboards and enclosed in a housing. In some embodiments, some of those components may be fabricated onto a single system-on-a-chip (SoC) (e.g., the SoC may include one or more electronic processing devices 802 and one or more storage devices 804). Additionally, in various embodiments, the computing device 800 may not include one or more of the components illustrated in FIG. 8, but may include interface circuitry for coupling to the one or more components using any suitable interface (e.g., a Universal Serial Bus (USB) interface, a High-Definition Multimedia Interface (HDMI) interface, a Controller Area Network (CAN) interface, a Serial Peripheral Interface (SPI) interface, an Ethernet interface, a wireless interface, or any other appropriate interface). For example, the computing device 800 may not include a display device 810, but may include display device interface circuitry (e.g., a connector and driver circuitry) to which an external display device 810 may be coupled.

The computing device 800 includes a processing device 802 (e.g., one or more processing devices). As used herein, the terms "electronic processor device" and "processing device" interchangeably refer to any device or portion of a device that processes electronic data from registers and/or memory to transform that electronic data into other electronic data that may be stored in registers and/or memory. In various embodiments, the processing device 802 may include one or more digital signal processors (DSPs), application-specific integrated circuits (ASICs), central processing units (CPUs), graphics processing units (GPUs), server processors, or any other suitable processing devices.

The computing device 800 also includes a storage device 804 (e.g., one or more storage devices). In various embodiments, the storage device 804 may include one or more memory devices, such as random-access memory (RAM) devices (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some embodiments, the storage device 804 may include memory that shares a die with the processing device 802. In such an embodiment, the memory may be used as cache memory and include embedded dynamic random-access memory (eDRAM) or spin transfer torque magnetic random-access memory (STT-MRAM), for example. In some embodiments, the storage device 804 may include non-transitory computer readable media having instructions thereon that, when executed by one or more processing devices (e.g., the processing device 802), cause the computing device 800 to perform any appropriate ones of the methods disclosed herein below or portions of such methods.

The computing device 800 further includes an interface device 806 (e.g., one or more interface devices 806). In various embodiments, the interface device 806 may include one or more communication chips, connectors, and/or other hardware and software to govern communications between the computing device 800 and other computing devices. For example, the interface device 806 may include circuitry for managing wireless communications for the transfer of data to and from the computing device 800. The term "wireless" and its derivatives may be used to describe circuits, devices, systems, methods, techniques, communications channels, etc., that may communicate data via modulated electromagnetic radiation through a nonsolid medium. The term does not imply that the associated devices do not contain any wires, although in some embodiments they might not. Circuitry included in the interface device 806 for managing wireless communications may implement any of a number of wireless standards or protocols, including but not limited to Institute for Electrical and Electronic Engineers (IEEE) standards including Wi-Fi (IEEE 802.11 family), IEEE 802.16 standards, Long-Term Evolution (LTE) project along with any amendments, updates, and/or revisions (e.g., advanced LTE project, ultramobile broadband (UMB) project (also referred to as "3GPP2"), etc.). In some embodiments, circuitry included in the interface device 806 for managing wireless communications may operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In some embodiments, circuitry included in the interface device 806 for managing wireless communications may operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In some embodiments, circuitry included in the interface device 806 for managing wireless communications may operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In some embodiments, the interface device 806 may include one or more antennas (e.g., one or more antenna arrays) configured to receive and/or transmit wireless signals.

In some embodiments, the interface device 806 may include circuitry for managing wired communications, such as electrical, optical, or any other suitable communication protocols. For example, the interface device 806 may include circuitry to support communications in accordance with Ethernet technologies. In some embodiments, the interface device 806 may support both wireless and wired communication, and/or may support multiple wired communication protocols and/or multiple wireless communication protocols. For example, a first set of circuitry of the interface device 806 may be dedicated to shorter-range wireless communications such as Wi-Fi or Bluetooth, and a second set of circuitry of the interface device 806 may be dedicated to longer-range wireless communications such as global positioning system (GPS), EDGE, GPRS, CDMA, WiMAX, LTE, EV-DO, or others. In some other embodiments, a first set of circuitry of the interface device 806 may be dedicated to wireless communications, and a second set of circuitry of the interface device 806 may be dedicated to wired communications.

The computing device 800 also includes battery/power circuitry 808. In various embodiments, the battery/power circuitry 808 may include one or more energy storage devices (e.g., batteries or capacitors) and/or circuitry for coupling components of the computing device 800 to an energy source separate from the computing device 800 (e.g., to AC line power).

The computing device 800 also includes a display device 810 (e.g., one or multiple individual display devices). In various embodiments, the display device 810 may include any visual indicators, such as a heads-up display, a computer monitor, a projector, a touchscreen display, a liquid crystal display (LCD), a light-emitting diode display, or a flat panel display.

The computing device 800 also includes additional input/output (I/O) devices 812. In various embodiments, the I/O devices 812 may include one or more data/signal transfer interfaces, audio I/O devices (e.g., microphones or microphone arrays, speakers, headsets, earbuds, alarms, etc.), audio codecs, video codecs, printers, sensors (e.g., thermocouples or other temperature sensors, humidity sensors, pressure sensors, vibration sensors, etc.), image capture devices (e.g., one or more cameras), human interface devices (e.g., keyboards, cursor control devices, such as a mouse, a stylus, a trackball, or a touchpad), etc.

Depending on the specific embodiment, various components of the interface devices 806 and/or I/O devices 812 can be configured to output suitable control signals, receive suitable control/telemetry signals, and receive and transmit data streams. In some examples, the interface devices 806 and/or I/O devices 812 include one or more analog-to-digital converters (ADCs) for transforming received analog signals into a digital form suitable for operations performed by the processing device 802 and/or the storage device 804. In some additional examples, the interface devices 806 and/or I/O devices 812 include one or more digital-to-analog converters (DACs) for transforming digital signals provided by the processing device 802 and/or the storage device 804 into an analog form suitable for being transmitted through a communication channel.

FIG. 9 is a block diagram illustrating an MS instrument support system 900 in which some or all of the scientific instrument support methods disclosed herein may be performed according to some examples. Various MS instrument support modules and methods disclosed herein (e.g., the system 100, the workflow 400, the process 500, and/or the method 600) may be implemented by one or more of an MS instrument 910, a user local computing device 920, a service computing device 930, and a remote computing device 940 of the MS instrument support system 900.

Any of the MS instrument 910, the user local computing device 920, the service computing device 930, or the remote computing device 940 may include any of the embodiments of the computing device 800 described above in reference to FIG. 8, and any of the MS instrument 910, the user local computing device 920, the service computing device 930, or the remote computing device 940 may take the form of any appropriate ones of the embodiments of the computing device 800.

The scientific instrument 910, the user local computing device 920, the service computing device 930, and/or the remote computing device 940 may each include a respective processing device 802, a respective storage device 804, and a respective interface device 806. The processing device 802 may take any suitable form, including the form of any of the processing devices 802 discussed herein with reference to FIG. 8, and the processing devices 802 included in different ones of the scientific instrument 910, the user local computing device 920, the service computing device 930, or the remote computing device 940 may take the same form or different forms. The storage device 804 may take any suitable form, including the form of any of the storage devices 804 discussed herein with reference to FIG. 8, and the storage devices 804 included in different ones of the scientific instrument 910, the user local computing device 920, the service computing device 930, or the remote computing device 940 may take the same form or different forms. The interface device 806 may take any suitable form, including the form of any of the interface devices 806 discussed herein with reference to FIG. 8, and the interface devices 806 included in different ones of the scientific instrument 910, the user local computing device 920, the service computing device 930, or the remote computing device 940 may take the same form or different forms.

The MS instrument 910, the user local computing device 920, the service computing device 930, and the remote computing device 940 may be in communication with other elements of the MS instrument support system 900 via communication pathways 908. The communication pathways 908 may communicatively couple the interface devices 806 of different ones of the elements of the MS instrument support system 900, as shown, and may be wired or wireless communication pathways (e.g., in accordance with any of the communication techniques discussed herein with reference to the interface devices 806 of the computing device 800 of FIG. 8). The particular MS instrument support system 900 depicted in FIG. 9 includes communication pathways between each pair of the scientific instrument 910, the user local computing device 920, the service computing device 930, and the remote computing device 940, but this "fully connected" implementation is purely illustrative, and in various embodiments, various ones of the communication pathways 908 may be absent. For example, in some embodiments, the service computing device 930 may not have a direct communication pathway 908 between its interface device 806 and the interface device 806 of the MS instrument 910 but may instead communicate with the MS instrument 910 via the communication pathway 908 between the service computing device 930 and the user local computing device 920 and the communication pathway 908 between the user local computing device 920 and the MS instrument 910. The MS instrument 910 may be included into a more-general and/or more-versatile scientific instrument.

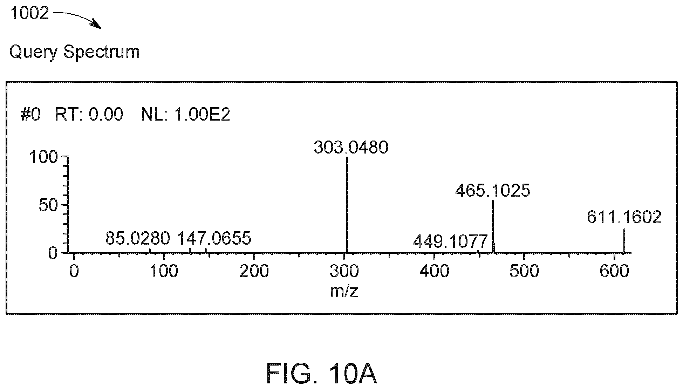

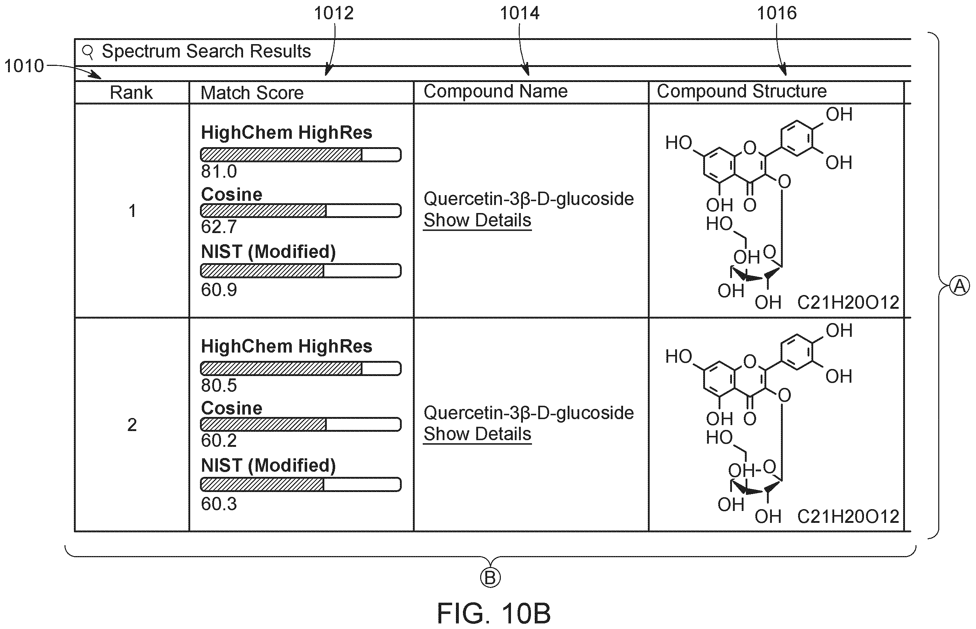

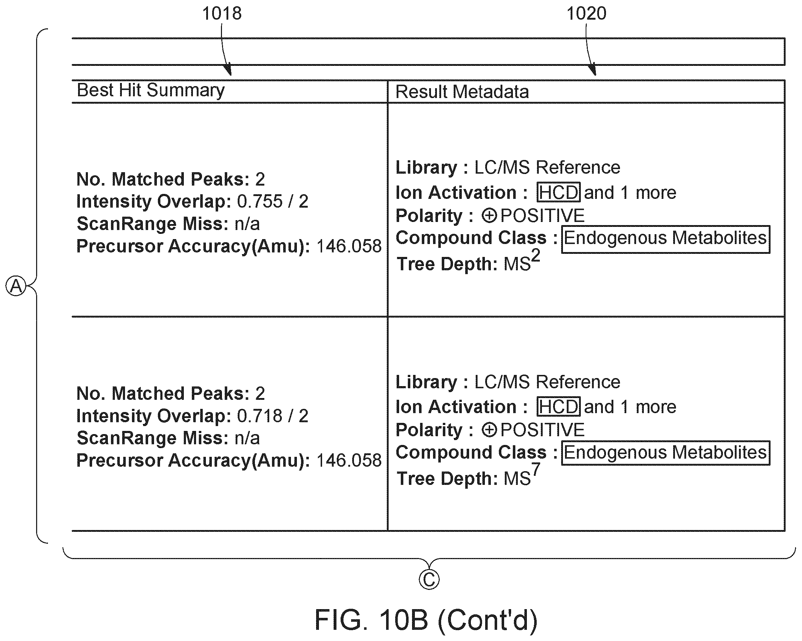

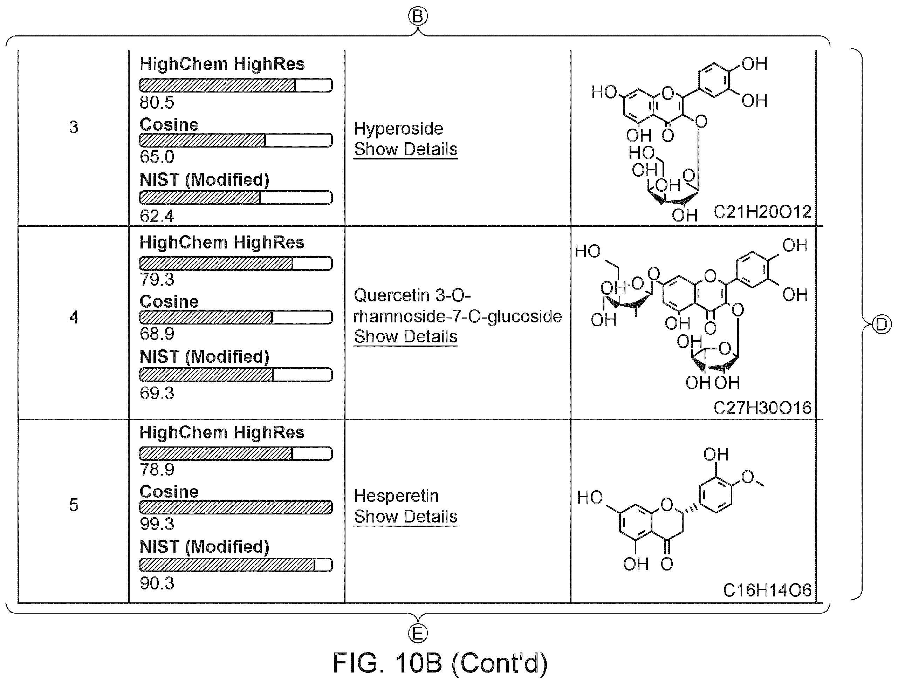

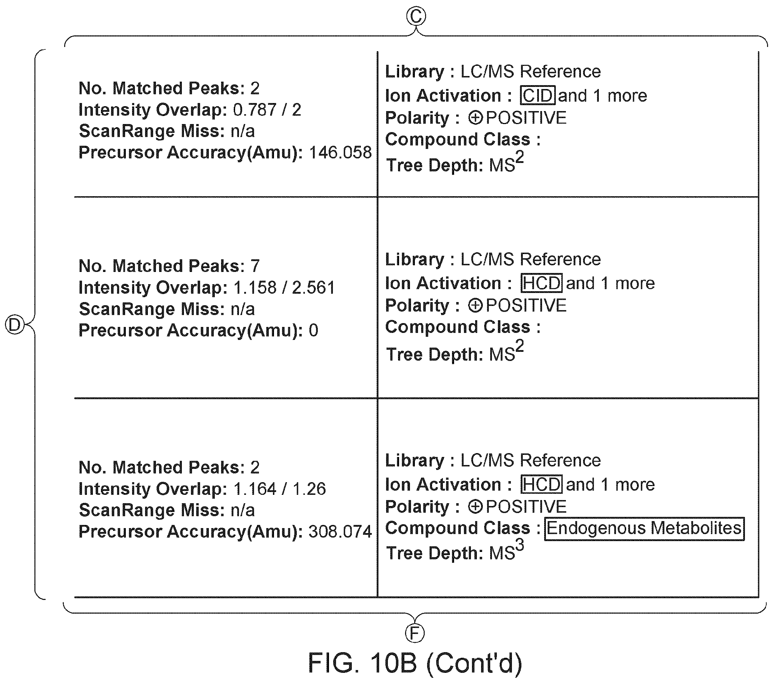

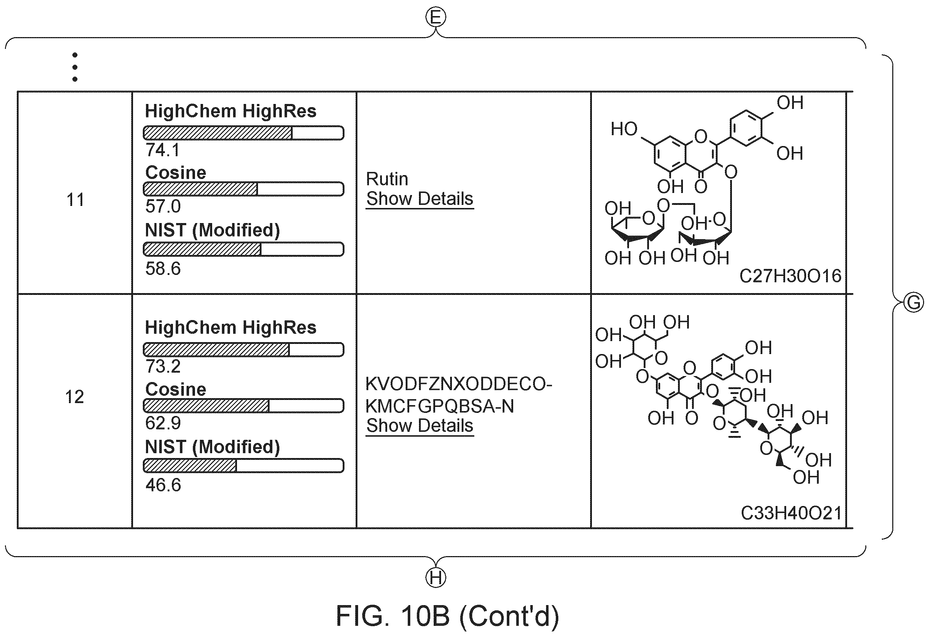

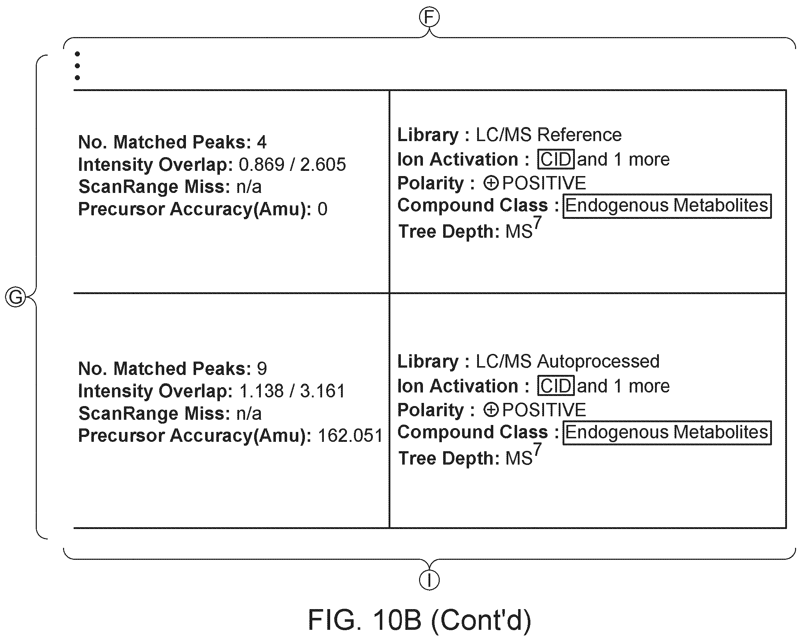

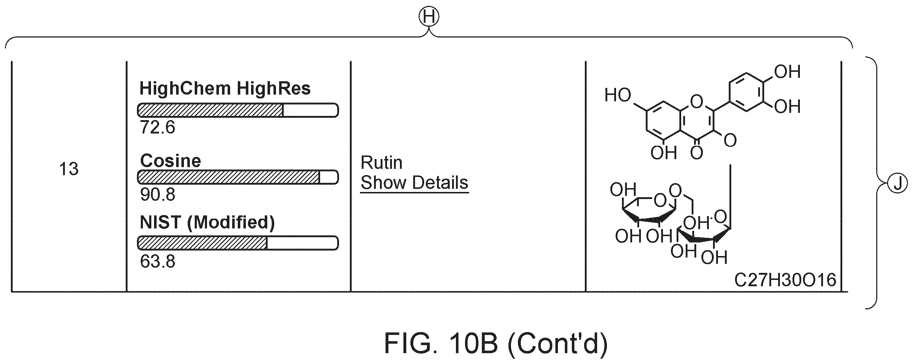

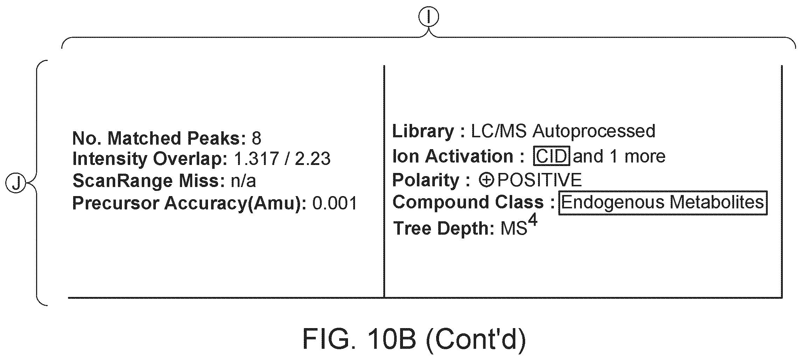

FIGS. 10A-10B illustrate improvements achievable with the method 600 over conventional analyte identification methods according to one example. More specifically, FIG. 10A graphically illustrates a query spectrum 1002 corresponding to an example control compound (Rutin, in this case) used to comparatively evaluate the relative performance of several analyte identification methods. FIG. 10B shows a table containing a ranked list of library hits obtained for the query spectrum 1002. The shown table has six columns that are labeled 1010-1020, respectively. The column 1010 displays the rank of the entries. The column 1012 displays the match scores for the three indicated algorithms. The column 1014 displays the compound names from the library. The column 1016 displays the compound structures. The column 1018 displays the hit summaries. The column 1020 displays the hit metadata from the library. The entries are ranked based on their HighChem HighRes scores.

The top five hits are characterized by relatively high similarity scores. However, none of the top twelve hits is the hit on the correct actual compound (Rutin), which only appears at the thirteen's position. Although each of the top three hits has HighChem HighRes score above 80, a closer look at the hit summaries (the column 1018) reveals that the number of matching peaks between the query spectrum 1002 and the corresponding library spectra is relatively low, at two. Additionally, the intensity of peaks does not match sufficiently accurately, as indicated by some of the metadata (the column 1020), such as the collision energy levels. In contrast, according to various embodiments, the ML model 130 is trained to learn the pertinent metadata and parameters to properly re-rank the library hits such that the correct hit gets pushed closer to the top of the hit list. In the example shown, a properly trained embodiment of the ML model 130 will change the rank of Rutin from the number 13 indicated in the table of FIG. 10B to a number within at least the top five hits. In other words, example embodiments disclosed herein will enable a significant reduction in the occurrence of false positives in the compound identification process.

According to one example disclosed above, e.g., in the summary section and/or in reference to any one or any combination of some or all of FIGS. 1-10, provided is a method performed via a computing device for providing support to a mass spectrometry (MS) system, the method comprising: obtaining from a mass spectral library (i) an ordered hitlist of reference spectra corresponding to a set of fragmentation spectra of an analyte acquired with the MS system; and (ii) a first set of metadata corresponding to the ordered hitlist of reference spectra; obtaining from the MS system a second set of metadata corresponding to the set of fragmentation spectra; and evaluating an order of entries in the ordered hitlist with a machine learning (ML) model based on the first set of metadata and the second set of metadata.

In some examples of the above method, obtaining the ordered hitlist includes submitting a search query with the set of fragmentation spectra to the mass spectral library.

In some examples of any of the above methods, at least one of the first and second sets of metadata includes a respective set of one or more parameters selected from the group consisting of: a normalized or absolute collision energy; one or more parameters of an ion activation method; a number of candidate compounds; one or more values of a matching score; an identity of a precursor ion; a number of peaks in a spectrum; a sparseness measure; an intensity of a peak and a corresponding accuracy; one or more distances between peaks in a spectrum; one or more spectrum labels; and a mean or average value of a selected numerical characteristic.

In some examples of any of the above methods, the matching score is computed using a dot product of a corresponding pair of spectra.

In some examples of any of the above methods, the ordered hitlist is ordered in a descending order of matching scores of the entries.

In some examples of any of the above methods, the ML model includes a model selected from the group consisting of: a random forest classifier; a gradient boosting model; a k-nearest-neighbors algorithm; and a variational autoencoder.

In some examples of any of the above methods, the evaluating comprises: with an encoder, generating a features vector based on the set of fragmentation spectra of the analyte, the reference spectra from the ordered hitlist, the first set of metadata, and the second set of metadata; and applying the features vector to the ML model.

In some examples of any of the above methods, the ML model is configured to change the order of the entries.

In some examples of any of the above methods, the method further comprises displaying, on a display device, a modified hitlist having the changed order of the entries.

In some examples of any of the above methods, the ML model is configured to determine one or more of: an estimated probability of the analyte belonging to a specified compound class; an estimated probability of the analyte belonging to a specified chemical class; and an estimated probability of the analyte being from a same compound class as a compound corresponding to a selected reference spectrum from the ordered hitlist.

In some examples of any of the above methods, at least one of the estimated probabilities differs from a corresponding probability determined at the mass spectral library.

In some examples of any of the above methods, the ML model is configured to determine an adjustment value to a matching score value provided by the mass spectral library with a respective reference spectrum of the ordered hitlist.

A non-transitory computer-readable medium storing instructions that, when executed by the computing device, cause the computing device to perform operations comprising any one of the above methods.

According to one example disclosed above, e.g., in the summary section and/or in reference to any one or any combination of some or all of FIGS. 1-10, provided is an apparatus for providing support to a mass spectrometry (MS) system, the apparatus comprising: an interface device; a processing device; and a memory device including program code, wherein the memory device and the program code are configured to, with the interface device and the processing device, cause the apparatus at least to: obtain from a mass spectral library (i) an ordered hitlist of reference spectra corresponding to a set of fragmentation spectra of an analyte acquired with the MS system; and (ii) a first set of metadata corresponding to the ordered hitlist of reference spectra; obtain from the MS system a second set of metadata corresponding to the set of fragmentation spectra; and evaluate an order of entries in the ordered hitlist with a machine learning (ML) model based on the first set of metadata and the second set of metadata.

In some examples of the above apparatus, at least one of the first and second sets of metadata includes a respective set of one or more parameters selected from the group consisting of: a normalized or absolute collision energy; one or more parameters of an ion activation method; a number of candidate compounds; one or more values of a matching score; an identity of a precursor ion; a number of peaks in a spectrum; a sparseness measure; an intensity of a peak and a corresponding accuracy; one or more distances between peaks in a spectrum; one or more spectrum labels; and a mean or average value of a selected numerical characteristic.

In some examples of any of the above apparatus, the ML model includes a model selected from the group consisting of: a random forest classifier; a gradient boosting model; a k-nearest-neighbors algorithm; and a variational autoencoder.

In some examples of any of the above apparatus, the memory device and the program code are further configured to, with the interface device and the processing device, cause the apparatus to: with an encoder, generate a features vector based on the set of fragmentation spectra of the analyte, the reference spectra from the ordered hitlist, the first set of metadata, and the second set of metadata; and apply the features vector to the ML model.

In some examples of any of the above apparatus, the ML model is configured to change the order of the entries.

In some examples of any of the above apparatus, the memory device and the program code are further configured to, with the interface device and the processing device, cause the apparatus to display, on a display device, a modified hitlist having the changed order of the entries.

In some examples of any of the above apparatus, the ML model is configured to determine one or more of: an estimated probability of the analyte belonging to a specified compound class; an estimated probability of the analyte belonging to a specified chemical class; and an estimated probability of the analyte being from a same compound class as a compound corresponding to a selected reference spectrum from the ordered hitlist.

It is to be understood that the above description is intended to be illustrative and not restrictive. Many implementations and applications other than the examples provided would be apparent upon reading the above description. The scope should be determined, not with reference to the above description, but should instead be determined with reference to the appended claims, along with the full scope of equivalents to which such claims are entitled. It is anticipated and intended that future developments will occur in the technologies discussed herein, and that the disclosed systems and methods will be incorporated into such future examples. In sum, it should be understood that the application is capable of modification and variation.

All terms used in the claims are intended to be given their broadest reasonable constructions and their ordinary meanings as understood by those knowledgeable in the technologies described herein unless an explicit indication to the contrary is made herein. In particular, use of the singular articles such as "a," "the," "said," etc. should be read to recite one or more of the indicated elements unless a claim recites an explicit limitation to the contrary.

The Abstract is provided to allow the reader to quickly ascertain the nature of the technical disclosure. It is submitted with the understanding that it will not be used to interpret or limit the scope or meaning of the claims. In addition, in the foregoing Detailed Description, it can be seen that various features are grouped together in various examples for the purpose of streamlining the disclosure. This method of disclosure is not to be interpreted as reflecting an intention that the claimed subject matter incorporate more features than are expressly recited in each claim. Rather, as the following claims reflect, inventive subject matter lies in fewer than all features of a single disclosed example. Thus, the following claims are hereby incorporated into the Detailed Description, with each claim standing on its own as a separately claimed subject matter.

Unless explicitly stated otherwise, each numerical value and range should be interpreted as being approximate as if the word "about" or "approximately" preceded the value or range.

Although the elements in the following method claims, if any, are recited in a particular sequence with corresponding labeling, unless the claim recitations otherwise imply a particular sequence for implementing some or all of those elements, those elements are not necessarily intended to be limited to being implemented in that particular sequence.

Unless otherwise specified herein, the use of the ordinal adjectives "first," "second," "third," etc., to refer to an object of a plurality of like objects merely indicates that different instances of such like objects are being referred to, and is not intended to imply that the like objects so referred-to have to be in a corresponding order or sequence, either temporally, spatially, in ranking, or in any other manner.

Unless otherwise specified herein, in addition to its plain meaning, the conjunction "if" may also or alternatively be construed to mean "when" or "upon" or "in response to determining" or "in response to detecting," which construal may depend on the corresponding specific context. For example, the phrase "if it is determined" or "if [a stated condition] is detected" may be construed to mean "upon determining" or "in response to determining" or "upon detecting [the stated condition or event]" or "in response to detecting [the stated condition or event]."

Also, for purposes of this description, the terms "couple," "coupling," "coupled," "connect," "connecting," or "connected" refer to any manner known in the art or later developed in which energy is allowed to be transferred between two or more elements, and the interposition of one or more additional elements is contemplated, although not required. Conversely, the terms "directly coupled," "directly connected," etc., imply the absence of such additional elements.

The functions of the various elements shown in the figures, including any functional blocks labeled as "processors" and/or "controllers," may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" or "controller" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and nonvolatile storage. Other hardware, conventional and/or custom, may also be included. Similarly, any switches shown in the figures are conceptual only. Their function may be carried out through the operation of program logic, through dedicated logic, through the interaction of program control and dedicated logic, or even manually, the particular technique being selectable by the implementer as more specifically understood from the context.

As used in this application, the terms "circuit," "circuitry" may refer to one or more or all of the following: (a) hardware-only circuit implementations (such as implementations in only analog and/or digital circuitry); (b) combinations of hardware circuits and software, such as (as applicable): (i) a combination of analog and/or digital hardware circuit(s) with software/firmware and (ii) any portions of hardware processor(s) with software (including digital signal processor(s)), software, and memory(ies) that work together to cause an apparatus, such as a mobile phone or server, to perform various functions); and (c) hardware circuit(s) and or processor(s), such as a microprocessor(s) or a portion of a microprocessor(s), that requires software (e.g., firmware) for operation, but the software may not be present when it is not needed for operation." This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in server, a cellular network device, or other computing or network device.

It should be appreciated by those of ordinary skill in the art that any block diagrams herein represent conceptual views of illustrative circuitry embodying the principles of the disclosure. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

## Claims

1. A method performed via a computing device for providing support to a mass spectrometry (MS) system, the method comprising:
obtaining from a mass spectral library:
an ordered hitlist of reference spectra corresponding to a set of fragmentation spectra of an analyte acquired with the MS system; and
a first set of metadata corresponding to the ordered hitlist of reference spectra;
obtaining from the MS system a second set of metadata corresponding to the set of fragmentation spectra; and
evaluating an order of entries in the ordered hitlist with a machine learning (ML) model based on the first set of metadata and the second set of metadata.

2. The method of claim 1, wherein obtaining the ordered hitlist includes submitting a search query with the set of fragmentation spectra to the mass spectral library.

3. The method of any of claims 1-2, wherein at least one of the first and second sets of metadata includes a respective set of one or more parameters selected from the group consisting of:
a normalized or absolute collision energy;
one or more parameters of an ion activation method;
a number of candidate compounds;
one or more values of a matching score;
an identity of a precursor ion;
a number of peaks in a spectrum;
a sparseness measure;
an intensity of a peak and a corresponding accuracy;
one or more distances between peaks in a spectrum;
one or more spectrum labels; and
a mean or average value of a selected numerical characteristic.

4. The method of claim 3, wherein the matching score is computed using a dot product of a corresponding pair of spectra; and
optionally wherein the ordered hitlist is ordered in a descending order of matching scores of the entries.

5. The method of any of claims 1-4, wherein the ML model includes a model selected from the group consisting of:
a random forest classifier;
a gradient boosting model;
a k-nearest neighbors algorithm; and
a variational autoencoder.

6. The method of any of claims 1-5, wherein the evaluating comprises:
with an encoder, generating a features vector based on the set of fragmentation spectra of the analyte, the reference spectra from the ordered hitlist, the first set of metadata, and the second set of metadata; and
applying the features vector to the ML model; and
optionally wherein the ML model is configured to change the order of the entries; and
further optionally comprising displaying, on a display device, a modified hitlist having the changed order of the entries.

7. The method of any of claims 1-6, wherein the ML model is configured to determine one or more of:
an estimated probability of the analyte belonging to a specified compound class;
an estimated probability of the analyte belonging to a specified chemical class; and
an estimated probability of the analyte being from a same compound class as a compound corresponding to a selected reference spectrum from the ordered hitlist.

8. The method of claim 7, wherein at least one of the estimated probabilities differs from a corresponding probability determined at the mass spectral library; and
optionally wherein the ML model is configured to determine an adjustment value to a matching score value provided by the mass spectral library with a respective reference spectrum of the ordered hitlist.

9. A non-transitory computer-readable medium storing instructions that, when executed by the computing device, cause the computing device to perform operations comprising the method of any of claims 1-8.

10. An apparatus for providing support to a mass spectrometry (MS) system, the apparatus comprising:
an interface device;
a processing device; and
a memory device including program code,
wherein the memory device and the program code are configured to, with the interface device and the processing device, cause the apparatus at least to:
obtain from a mass spectral library:
an ordered hitlist of reference spectra corresponding to a set of fragmentation spectra of an analyte acquired with the MS system; and
a first set of metadata corresponding to the ordered hitlist of reference spectra;
obtain from the MS system a second set of metadata corresponding to the set of fragmentation spectra; and
evaluate an order of entries in the ordered hitlist with a machine learning (ML) model based on the first set of metadata and the second set of metadata.

11. The apparatus of claim 10, wherein at least one of the first and second sets of metadata includes a respective set of one or more parameters selected from the group consisting of:
a normalized or absolute collision energy;
one or more parameters of an ion activation method;
a number of candidate compounds;
one or more values of a matching score;
an identity of a precursor ion;
a number of peaks in a spectrum;
a sparseness measure;
an intensity of a peak and a corresponding accuracy;
one or more distances between peaks in a spectrum;
one or more spectrum labels; and
a mean or average value of a selected numerical characteristic.

12. The apparatus of any of claims 10-11, wherein the ML model includes a model selected from the group consisting of:
a random forest classifier;
a gradient boosting model;
a k-nearest neighbors algorithm; and
a variational autoencoder.

13. The apparatus of any of claims 10-12, wherein the memory device and the program code are further configured to, with the interface device and the processing device, cause the apparatus to:
with an encoder, generate a features vector based on the set of fragmentation spectra of the analyte, the reference spectra from the ordered hitlist, the first set of metadata, and the second set of metadata; and
apply the features vector to the ML model.

14. The apparatus of claim 13, wherein the ML model is configured to change the order of the entries; and
optionally wherein the memory device and the program code are further configured to, with the interface device and the processing device, cause the apparatus to display, on a display device, a modified hitlist having the changed order of the entries.

15. The apparatus of any of claims 10-14, wherein the ML model is configured to determine one or more of:
an estimated probability of the analyte belonging to a specified compound class;
an estimated probability of the analyte belonging to a specified chemical class; and
an estimated probability of the analyte being from a same compound class as a compound corresponding to a selected reference spectrum from the ordered hitlist.
